Europäisches Patentamt

European Patent Office

Office européen des brevets

(11) Veröffentlichungsnummer : **0 117 972**
**B1**

# (12) EUROPÄISCHE PATENTSCHRIFT

(45) Veröffentlichungstag der Patentschrift :
19.08.87

(21) Anmeldenummer : 84100159.7

(22) Anmeldetag : 09.01.84

(51) Int. Cl.⁴ : **A 61 N   1/05**, A 61 N   1/362

(54) Indifferente Elektrode für ein Herzschrittmachersystem.

(30) Priorität : 11.01.83 DE 3300672

(43) Veröffentlichungstag der Anmeldung :
12.09.84 Patentblatt 84/37

(45) Bekanntmachung des Hinweises auf die Patenterteilung : 19.08.87 Patentblatt 87/34

(84) Benannte Vertragsstaaten :
DE FR IT NL

(56) Entgegenhaltungen :
EP-A- 0 054 781
DE-A- 2 165 622
DE-A- 2 613 072
DE-A- 2 638 563
DE-A- 2 702 240
DE-A- 2 706 636
DE-A- 2 842 318
DE-A- 2 922 354
US-A- 4 281 669
BIOMEDIZINISCHE TECHNIK, Band 25, Nr. 7, 8,
Juli/August 1980, Berlin H.J. BISPING "Neue Schrittmachersonden - ein Bericht aus Montreal" Seiten
170-175

(73) Patentinhaber : **Siemens Aktiengesellschaft Berlin
und München**
**Wittelsbacherplatz 2**
**D-8000 München 2 (DE)**

(72) Erfinder : **Elmqvist, Hakan, Dr.**
**Sunnedalsvaegen 7**
**S-161 38 Bromma (SE)**
Erfinder : **Mund, Konrad, Dr.**
**Langenbrucker Weg 6**
**D-8521 Uttenreuth (DE)**

## Beschreibung

Die Erfindung betrifft ein Herzschrittmachersystem mit mindestens einer aktiven und einer indifferenten Elektrode, wobei diese durch die Oberfläche des Herzschrittmachergehäuses gebildet ist. Derartige Herzschrittmachersysteme werden u. a. zur bifokalen Stimulierung benutzt und weisen beispielsweise eine aktive Elektrode auf, die nach der Implantation des Herzschrittmachers in die Vorkammer des Herzens geführt ist und eine weitere aktive Elektrode, die in die Herzkammer geführt ist. An derartige implantierbare Reizelektroden, die allgemein aus einem isolierten Leiter und einem mit diesem Leiter verbundenen Elektrodenkopf mit dem aktiven Bereich bestehen, werden im wesentlichen zwei Forderungen gestellt :

1. Das Elektrodenmaterial muss körperverträglich sein, so dass die Bildung von Bindegewebsschichten mit einer Dicke grösser 100 um unterbunden wird, damit die Reizschwelle weitgehend konstant bleibt.

2. An der Phasengrenze Elektrode/Körperflüssigkeit soll sich eine hohe Doppelschichtkapazität ausbilden, so dass der Polarisationsanstieg während der Reizimpulse (0,5 bis 1 ms, 1 Hz, 10 mA, 10 mm$^2$ kleiner als 0,1 V bleibt.

Diese Forderungen werden in besonders hohem Masse von Elektroden erfüllt, bei denen der aktive Bereich aus Glaskohlenstoff besteht (s. DE-OS 2613072). Hierbei wird eine hohe Doppelschichtkapazität von bis zu 0,1 F/cm$^2$ durch eine Aktivierung der Oberfläche des Glaskohlenstoffes erreicht.

An die indifferente Elektrode wurde bisher lediglich die Forderung nach Körperverträglichkeit gestellt.

Bei den genannten Herzschrittmachersystemen mit zwei aktiven Elektroden und einer gemeinsamen indifferenten Elektrode können unter Umständen aufgrund von Wechselwirkungen zwischen den Elektroden Probleme entstehen. Die Ursache dazu ist ein Polarisationsanstieg an der indifferenten Elektrode bei der Stimulierung mit einer aktiven Elektrode. Diese Polarisation baut sich nur langsam wieder ab und beeinflusst nachteilig die Möglichkeit, die andere aktive Elektrode zur Detektierung von Herzdepolarisationen während dieser Zeit auszunützen, da die Polarisation eine Erhöhung der elektrochemischen Impedanz des Systemes darstellt, die das Erfassen der sehr kleinen Mesströme erheblich erschwert.

Weiterhin kann die Funktionsfähigkeit des Herzschrittmachersystemes durch Muskelzuckungen beeinträchtigt werden. Diese Muskelzuckungen beruhen im allgemeinen darauf, dass die Stimulierungsimpulse nicht nur den Herzmuskel, sondern auch reizbares Gewebe in der Nähe des Herzschrittmachergehäuses, dass die indifferente Elektrode in dem Elektrodensystem darstellt, stimuliert. Die mit diesen Muskelzuckungen verbundenen elektrischen Spannungsimpulse können unter Umständen nicht vorhandene Herzaktivitäten vortäuschen. Bisher wurde versucht, diese Gefahr zu beseitigen, indem der Herzschrittmacher mit einer isolierenden Hülle umgeben wurde. In dieser Hülle befindet sich ein Loch, durch das der Strom passieren kann (SIEMENS-ELEMA Prospekt ME 372/5406.101, 1979). Der Herzschrittmacher wird dann so implantiert, dass sich das Loch auf der dem reizbaren Gewebe abgewendeten Seite befindet. Im allgemeinen hören damit die Muskelzuckungen auf.

Neben diesen Muskelzuckungen bestand weiterhin das Problem, dass der Herzschrittmacher Myopotentiale der Skelettmuskulatur in der Nähe des Herzschrittmachers als Depolarisationen der Herzmuskulatur detektieren konnte. Auch hierdurch bestand wieder die Gefahr einer Beeinträchtigung der Herzschrittmacherfunktion. Auch dieses Problem liess sich durch die isolierende Hülle und die richtige Plazierung des Herzschrittmachers bei der Implantation lösen.

Diese isolierende Hülle weist aber auch Nachteile auf. Zum einen vermindert sich die Oberfläche der indifferenten Elektrode, wodurch die Polarisationseffekte ansteigen und die Effektivität der Stimulierungsimpulse und die Empfindlichkeit des Herzschrittmachers zum Detektieren von Herzaktivitäten herabgesetzt wird. Zum anderen ergeben sich hinsichtlich der Herstellung, der Hygiene und der Zuverlässigkeit Nachteile durch diese normalerweise aus irgendeinem organischen Material bestehende isolierende Hülle.

Der vorliegenden Erfindung liegt daher die Aufgabe zugrunde, die bislang bei Herzschrittmachersystemen der eingangs genannten Art durch die indifferente Elektrode hervorgerufenen Probleme zu vermeiden oder zumindest wesentlich zu vermindern und gleichzeitig die Empfindlichkeit des Elektrodensystemes zu erhöhen.

Diese Aufgabe wird erfindungsgemäss dadurch gelöst, dass ein Teil dieser Oberfläche eine Oberflächenschicht aufweist, die an der Phasengrenze zur Körperflüssigkeit eine hohe Doppelschichtkapazität besitzt. Dadurch wird beispielsweise erreicht, dass der Polarisationsanstieg bei der Stimulierung sehr gering ist. Bei einem bifokalen System kann daher mit einer Elektrode stimuliert und praktisch ohne Empfindlichkeitsverlust mit der anderen Elektrode detektiert werden.

Weiterhin ist es damit möglich, das Herzschrittmachergehäuse als indifferente Elektrode insgesamt leitfähig zu belassen, d. h. von einer isolierenden Hülle abzusehen. Die bisher von der Isolierung freigelassene Fläche oder eine entsprechende wird mit der erfindungsgemässen Oberflächenschicht versehen und weist daher gegenüber dem umgebenden Gewebe eine wesentlich niedrigere elektrochemische Impedanz auf als die restliche Oberfläche des Herzschrittmachergehäuses. Dadurch erhält man eine Spannungs- respektive Stromteilung. Den Bereich mit der erfindungsgemässen Oberflächenschicht wird man bei der Implantation wiederum von dem reizbaren Gewebe oder von Myopotentiale erzeugenden Geweben abwenden.

Besonders vorteilhaft für das Herzschrittmachersystem ist es beispielsweise, wenn die Reiz- und die indifferente Elektrode aus demselben Material besteht, weil dann keine materialbedingten Potentialdifferenzen auftreten können.

In einem in-vitro-Experiment wurden dazu eine TiN-Reizelektrode und zwei aus Ti gefertigte Gehäusehälften des Schrittmachers in einen mit 0,035 M NaCl gefüllten Elektrolyttrog so eingebaut, dass sie die Schrittmacheranordnung simulierten. Beide Gehäuseteile waren getrennt kontaktiert, und über Messwiderstände (152) konnten die Teilströme bei Belastung mit galvanostatischen Impulsen (I = 10 mA, 1 ms) verfolgt werden. Es zeigte sich, dass zu Beginn des Impulses die der Reizelektrode zugewandte Gehäusehälfte bevorzugt wird und dass nach ca. 0,4 ms die hintere Gehäusehälfte stärker belastet wird.

Im Vergleichsexperiment wurde das hintere Gehäuseteil mit einer 6 μm dicken Schicht aus porösem TiN versehen. Dabei zeigte sich, dass jetzt bereits nach 0,2 ms bevorzugt der Strom von der Rückseite getragen wird. Diese Seite soll also bei Implantation kein Muskelgewebe berühren.

Auf besonders einfache Art und Weise ergibt sich die erfindungsgemässe Oberflächenschicht, in dem man die leitfähige Oberfläche der indifferenten Elektrode, bei Herzschrittmachergehäusen handelt es sich beispielsweise um metallische Materialien wie Platin/Iridium, im aktiven Bereich aufrauht. Durch dieses Aufrauhen vergrössert sich quasi die Oberfläche um ein Vielfaches gegenüber einer glatten Oberflächenschicht, wodurch bereit eine erhebliche Steigerung der Doppelschichtkapazität erzielt wird. Eine weitere Steigerung der Doppelschichtkapazität erhält man dadurch, dass die Oberflächenschicht aus Aktivkohle — insbesondere aktiviertem Glaskohlenstoff — besteht, wie es bereits für die Elektrodenköpfe der Reizelektroden bekannt ist.

Eine besonders einfach herzustellende und mechanisch stabile Oberflächenschicht bildet eine poröse Schicht aus einem Carbid, Nitrid oder Carbonitrid wenigstens eines der Metalle Titan, Vanadium, Zirkonium, Niob, Molybdän, Hafnium, Tantal oder Wolfram.

Die die Carbide, Nitride und Carbonitride bildenden Metalle sind sämtlich Elemente der vierten bis sechsten Nebengruppe des Periodensystems und zählen somit zu den sogenannten Uebergangsmetallen. Carbide MeC und Nitride MeN der genannten Art (Me = Metall) sind beispielsweise TiC, TiN, ZrC, oder TaN. Die poröse Schicht ist dabei gut leitend und weist eine Dicke zwischen 1 und 100 μm auf. Dabei ergeben sich Doppelschichtkapazitäten die etwa in der gleichen Grössenordnung wie die von Aktivkohle liegen. Die Herstellung der Schichten ist jedoch erheblich einfacher. Als Trägermaterial für die Schichten dienen gewebefreundliche Metalle oder Metall-Legierungen wie beispielsweise Elgiloy oder Vorzugsweise Platin und Titan.

Um das Auftreten von Mischpotentialen zu vermeiden, kann in Weiterbildung der Erfindung vorgesehen sein, dass sich zwischen der porösen Schicht und dem Trägermaterial eine dichte Schicht befindet, die aus demselben Material besteht wie die poröse Schicht. Dadurch ist es unter Umständen auch möglich, als Trägermaterial ein nichtgewebefreundliches Material zu wählen, das zunächst mit einer dichten Schicht aus einem gewebefreundlichen Material umgeben wird, das dann wiederum zumindest im aktiven Bereich mit einer porösen Schicht dieses Materiales beschichtet wird. Die porösen Carbid-, Nitrid- oder Carbonitrid-Schichten werden vorzugsweise durch reaktives Ionenplattieren, d. h. durch physikalische. Dampfabscheidung, auf dem als Substrat dienenden Trägermaterial aufgebracht.

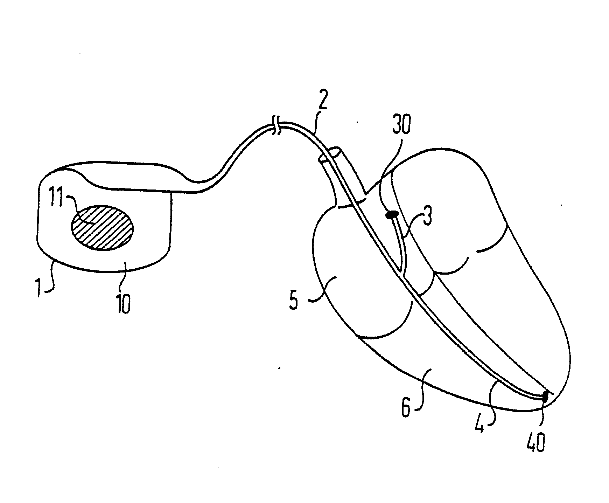

Anhand einer Figur wird im folgenden eine Ausführungsform des erfindungsgemässen Herzschrittmachersystemes näher beschrieben und erläutert. Die Figur zeigt dabei schematisch ein für die bifokale Stimulierung vorgesehenes System.

Das Herzschrittmachersystem besteht aus dem eigentlichen implantierbaren Herzschrittmacher 1 mit einem geschlossenen Gehäuse 10 beispielsweise aus Titan. An diesen Herzschrittmacher sei eine Elektrodenleitung 2 für zwei Stimulierungselektroden 3 und 4 angeschlossen. Die Stimulierungselektrode 3 weist einen Elektrodenkopf 30 auf, der für die Stimulierung und zum Abfühlen der Herzaktivitäten in der linken Vorkammer 5 eines Patienten vorgesehen ist. Die Elektrodenleitung 4 weist einen Elektrodenkopf 40 auf, der entsprechend für die Stimulierung und Detektierung von Herzaktivitäten in der linken Herzkammer 6 vorgesehen ist.

Wesentlich für dieses Herzschrittmachersystem ist nun, dass das Gehäuse 10 des Herzschrittmachers 1 einen Bereich 11 mit einer porösen Schicht aus beispielsweise Titannitrid aufweist. Durch diese Beschichtung werden gleichzeitig zwei Effekte möglich. Zum einen wird die Doppelschichtkapazität zwischen dem Gehäuse 10 und dem umgebenden nicht dargestellten Gewebe stark vergrössert, wodurch Polarisationseffekte vermieden werden. Zum anderen besteht die Möglichkeit, bei der Implantation das Gehäuse so anzuordnen, das die Schicht von reizbarem Muskelgewebe oder von Myopotentiale erzeugende Muskelgewebe abgewendet ist, so dass die daher rührenden elektrischen Impulse im wesentlichen unterdrückt werden und die Funktion des Herzschrittmachersystems nicht störend beeinflussen.

## Patentansprüche

1. Herzschrittmachersystem mit mindestens einer aktiven und einer indifferenten Elektrode, wobei diese durch die Oberfläche des Herzschrittmachergehäuses gebildet ist, dadurch gekennzeichnet, dass ein Teil (11) dieser Oberfläche (10) eine Oberflächenschicht aufweist, die an der Phasengrenze zur Körperflüssigkeit eine hohe Doppelschichtkapazität besitzt.

2. Herzschrittmachersystem nach Anspruch 1, dadurch gekennzeichnet, dass die Oberflächenschicht durch eine aufgerauhte Oberfläche der Elektrode (1) gebildet ist.

3. Herzschrittmachersystem nach Anspruch 1, dadurch gekennzeichnet, dass die Oberflächenschicht aus Aktivkohle — insbesondere aktiviertem Glaskohlenstoff — besteht.

4. Herzschrittmachersystem nach Anspruch 1, dadurch gekennzeichnet, dass die Oberflächenschicht aus einer porösen Schicht aus einem Carbid, Nitrid oder Carbonitrid wenigstens eines der Metalle Titan, Vanadium, Zirkonium, Niob, Molybdän, Hafnium, Tantal oder Wolfram besteht.

5. Herzschrittmachersystem nach einen der Ansprüche 1 bis 4, dadurch gekennzeichnet, dass die Oberflächenschicht eine Schichtdicke zwischen 2 und 100 μm, vorzugsweise zwischen 5 und 50 μm, aufweist.

6. Herzschrittmachersystem nach Anspruch 4, dadurch gekennzeichnet, dass das Trägermaterial für die Oberflächenschicht Titan oder Platin ist.

7. Herzschrittmachersystem nach Anspruch 6, dadurch gekennzeichnet, dass sich zwischen dem Trägermaterial und der porösen Schicht eine dichte Schicht aus einem entsprechenden Material wie die poröse Schicht befindet.

8. Herzschrittmachersystem nach Anspruch 7, dadurch gekennzeichnet, dass die dichte Schicht eine Schichtdicke zwischen 2 und 10 μm aufweist.

**Claims**

1. A heart pacemaker system comprising at least one active electrode and one inert electrode, the inert electrode being formed by the surface of the heart pacemaker housing, characterised in that a part (11) of this surface (10) is provided with a surface layer which has a high double layer capacitance at the phase boundary to the body fluid.

2. A heart pacemaker system as claimed in Claim 1, characterised in that the surface layer is formed by a roughened surface of the electrode (1).

3. A heart pacemaker system as claimed in Claim 1, characterised in that the surface layer consists of activated carbon, in particular, activated vitreous carbon.

4. A heart pacemaker system as claimed in Claim 1, characterised in that the surface layer consists of a porous layer made of a carbide, nitride or carbonitride of at least one of the metals titanium, vanadium, zirconium, niobium, molybdenum, hafnium, tantalum or tungsten.

5. A heart pacemaker system as claimed in one of the Claims 1 to 4, characterised in that the surface layer has a thickness of between 2 and 100 μm, preferably between 5 and 50 μm.

6. A heart pacemaker system as claimed in Claim 4, characterised in that the carrier material for the surface layer is titanium or platinum.

7. A heart pacemaker system as claimed in Claim 6, characterised in that a dense layer consisting of a material corresponding to that of the porous layer, is arranged between the carrier material and the porous layer.

8. A heart pacemaker system as claimed in Claim 7, characterised in that the dense layer has a thickness of between 2 and 10 μm.

**Revendications**

1. Système de stimulateur cardiaque, comprenant au moins une électrode active et au moins une électrode indifférente, celle-ci étant formée par la surface du boîtier du stimulateur cardiaque, caractérisé en ce qu'une partie (11) de cette surface (10) comprend une couche superficielle qui possède, à la limite des phases avec le liquide de l'organisme, une grande capacité à doubles couches.

2. Système de stimulateur cardiaque suivant la revendication 1, caractérisé en ce que la couche superficielle est formée par une surface rendue rugueuse de l'électrode (1).

3. Système de stimulateur cardiaque suivant la revendication 1, caractérisé en ce que la couche superficielle est en charbon actif, notamment en produit obtenu par décomposition thermique de composés aromatiques avec réticulation.

4. Système de stimulateur cardiaque suivant la revendication 1, caractérisé en ce que la couche superficielle est constituée d'une couche poreuse en carbure, nitrure ou carbonitrure, d'au moins l'un des métaux que sont le titane, le vanadium, le zirconium, le niobium, le molybdène, l'hafnium, le tantale ou le tungstène.

5. Système de stimulateur cardiaque suivant l'une des revendications 1 à 4, caractérisé en ce que la couche superficielle a une épaisseur comprise entre 2 et 100 microns et, de préférence, comprise entre 5 et 50 microns.

6. Système de stimulateur cardiaque suivant la revendication 4, caractérisé en ce que le matériau support de la couche superficielle est du titane ou du platine.

7. Système de stimulateur cardiaque suivant la revendication 6, caractérisé en ce qu'entre le matériau support et la couche poreuse est interposée une couche dense en un matériau correspondant à celui de la couche poreuse.

8. Système de stimulateur cardiaque suivant la revendication 7, caractérisé en ce que la couche dense a une épaisseur comprise entre 2 et 10 microns.